# EUROPEAN PATENT APPLICATION

(11) **EP 3 572 064 A1**
(43) Date of publication of application: **27.11.2019**
(21) Application number: 18380004.4
(22) Date of filing: 22.05.2018
(51) Int. Cl.: A61K 6/033, A61K 8/24, A61L 27/12

(54) **SHARK TEETH BIOCERAMICS AND USES THEREOF**

(71) Applicant: Universidade de Vigo, 36310 Vigo (ES)
(72) Inventor: Fernández, PÍO Manuel González, E36208 Vigo (ES); Rodríguez, Julia Serra, E36211 Vigo (ES); Álvarez, Miriam López, E36210 Vigo (ES); Valencia, Cosme Rodríguez, E15883 TEO, A Coruña (ES); Chiussi, Stefano, E36208 Vigo (ES); Senra, Estefanía López, E36317 Vigo (ES)

(57) **Abstract**

The present invention relates to bioceramics obtained from shark teeth, either from tooth enamel, dentine, or a mixture of both, for its use in the treatment of hard tissue injuries or pathologies. The invention can also be used in a dentifrice or mouthwash for the prevention of dental caries, remineralization of teeth or to inhibit dental sensitivity.

## Description

### Field of the Invention

The present invention relates to bioceramics useful for the treatment or prevention of hard tissue injuries or pathologies. The invention can also be used in a dentifrice or mouthwash, either to remineralize teeth or to inhibit dental sensitivity.

### Background of the Invention

Mineralized tissues are hybrid materials formed mainly by an organic matrix, water and mineral compounds. Typical mineralized tissues are bones and teeth, with an inorganic content ranging from 70 wt% in bones to 96 wt% in enamel teeth. The mineral phase of these tissues is a high strength and low weight ceramic material, mostly calcium phosphate compounds based on carbonated hydroxyapatite crystals with 10% calcium deficiency. These crystals are hexagonal or monoclinic prisms of nanometric size. Their calcium-deficient composition favors a high level of solubility and promotes constant bone regeneration through continuous dissolution and crystallization cycles. In human tooth enamel, the crystals contain large amounts of carbonate ions (∼ 2-5 wt%) and a variety of elements in trace concentrations, such as Cl, Mg, K, Fe, Se, Sr, Cr, Ni, Co, Ti, Al and F. Studies have demonstrated that the presence of some trace elements can influence the physical properties of the crystals. An interesting case is the shark tooth enameloid, which, with its high fluorine content of 3.1 wt% close to that of the geological fluorapatite single crystal (3.64 wt%) presents a high degree of hardness and stability.

Bone void fillers are currently highly demanded in orthopedical, dental and maxillofacial surgery for repairing, replacing or regenerating defects at human bones or teeth. Fillers are required in the reconstruction of missing bone cavities, bone atrophies or congenital malformations and to promote bone regeneration at damaged or injured tissues caused by various traumas. More specifically, in dental and maxillofacial applications, they are commonly used to promote periodontal regeneration, maxillary sinus elevation, to repair defects after teeth extraction and/or in cases of implants placement. Within all these materials, apatite in the form of hydroxyapatite (HA), Ca₁₀(PO₄)₆(OH)₂, is demanded to simulate the composition of the mineral part of the human bone.

The enameloid and dentine of shark teeth constitute two new direct sources of bioapatites with the incorporation of trace elements such as fluorine (F) (in the form of fluorapatite, Ca₅(PO₄)₃F) and Mg on their respective apatite-based structures. The latter is mainly dentine, together with small contributions from organic compounds and the hierarchical distribution of pores. The obtention of fluorapatite nanocristals from shark teeth has been disclosed in EP2853622-B1.

In view of an increasing demand for bioceramics that can be particularly useful as a hard tissue replacement material or in order to prevent hard tissue pathologies, it is necessary to ameliorate the bioceramics which contain a suitable proportion of apatite, such that they can give rise to biocompatible materials with improved healing or injury prevention capacity.

### Summary of the Invention

The authors of the present invention have surprisingly found that a bioceramic obtained from shark teeth is useful in the treatment or prevention of hard tissue injuries or pathologies.

Therefore, in a first aspect the invention relates to a bioceramic obtained from shark teeth for use in the treatment or prevention of a hard tissue injury or pathology.

In another aspect, the invention relates to a dentifrice, a gel or a mouthwash comprising a bioceramic according to the invention.

### Brief description of the figures

**Figure 1****.** Concentration of Ca, P, and Mg (mg/l) measured by means of ICP-OES corresponding to each collected volume after immersion of the different materials (bioceramic obtained from shark tooth enamel, from shark tooth dentine, a mixture of shark tooth, Bio-Oss® and Bi-Ostetic®) in the different citrate buffers (pH=4.0, 4.5, 5.0, and 6.0).
**Figure 2****.** X-ray images from the follow-up of the radiocarpal joint of a mongrel dog treated with the bioceramic obtained from shark tooth (*Prionace glauca or Isurus oxyrinchus).*

### Detailed Description of the Invention

### Bioceramic obtained from shark teeth for use in the treatment or prevention of a hard tissue injury or pathology

The inventors of the present invention have found that a bioceramic obtained from shark teeth is useful in the treatment of diseases which require remineralization of hard tissues. Accordingly, in one aspect, the invention relates to a bioceramic obtained from shark teeth for use in the treatment or prevention of a hard tissue injury or pathology.

As used herein "bioceramic" refers to a biomaterial that can be produced by sintering or melting inorganic raw materials to create an amorphous or a crystalline solid body or which can be isolated from natural sources. The "bioceramics" for use according to the present invention are biocompatible, understood as the ability of a material to perform with an appropriate host response in a specific situation. Bioceramics range in biocompatibility from the ceramic oxides, which are inert in the body, to the other extreme of resorbable materials, which are eventually replaced by the body after they have assisted repair. Bioceramics can be in many types of medical procedures, for example as rigid materials in surgical implants, though some bioceramics are flexible. Bioceramics are closely related to either the body's own materials or are extremely durable metal oxides. In particular amongst the components of ceramics are calcium, silica, phosphorous, magnesium, potassium, and sodium. Bioceramic used in the fabrication for the tissue engineering might be classified as nonresorbable (relatively inert), bioactive or surface active (semi-inert), and biodegradable or resorbable (non-inert). Alumina, zirconia, silicon nitride, and carbons are inert bioceramics. Certain glass ceramics are dense hydroxyapatites (HA, 9CaO Ca(OH)₂ 3P₂O₅), semi-inert (bioactive), and calcium phosphates, aluminum-calcium-phosphates, coralline, tricalcium phosphates (3CaO P₂O₅), zinc-calcium-phosphorous-oxides, zinc-sulfate-calcium-phosphates, ferric-calcium-phosphorous-oxides, and calcium aluminates are resorbable ceramics. Among these bioceramics, synthetic apatite and calcium phosphate minerals, coral-derived apatite, bioactive glass, and demineralized bone particle (DBP) are widely used in hard tissue engineering area. As disclosed herein, "biomaterial" is any substance that has been engineered to be able to interact with biological systems for a medical purpose - either a therapeutic (treat, augment, repair or replace a tissue function of the body), pathology preventive or a diagnostic one. The biomaterial can be obtained by any process known in the art.

As disclosed herein, the bioceramic is obtained from shark teeth or from the dentine or enameloid part thereof. In a preferred embodiment the bioceramic is obtained from teeth of the species *Prionace glauca.* In another preferred embodiment the bioceramic is obtained from teeth of the species *Isurus oxyrinchus.* In a more preferred embodiment, the bioceramic is obtained from a mixture of teeth of *Prionace glauca* and *Isurus oxyrinchus.*

In order to obtain the bioceramic for use according to the invention, shark teeth are treated as described (López-Álvarez et al, Biomed. Mater. 11 (2016) 035011) in order to separate the two sections of the teeth, the enameloid (the outer part of shark teeth) and the dentine (the inner part). As disclosed herein, the bioceramic can also be a mixture of both enameloid and dentine. The enameloid and dentine of shark teeth constitute two direct sources of bioapatites with the incorporation of trace elements such as fluorine (F), Magnesium (Mg) and Sodium (Na) or other trace elements in lower percentage, such as Potassium (K) or Strontium (Sr) amongst others on their respective apatite-based structures. In an embodiment, previous to the bioceramic isolation, sharks' teeth are washed and boiled in water to separate the organic remains of the sharks' teeth, and afterwards are dried in a laboratory oven. In a preferred embodiment, sharks' teeth are washed and boiled in water for 3h. In another preferred embodiment, sharks' teeth are dried in a laboratory oven at 60 °C for 24 h. In a more preferred embodiment, sharks' teeth are washed and boiled in water for 3h and dried in a laboratory oven at 60 °C for 24 h.

Shark teeth are composed of two sections: the enameloid and the dentine. As disclosed herein, "enameloid" or "enamel" is one of the four major tissues that make up the tooth in humans and many other animals, including some species of fish such as sharks. It makes up the normally visible part of the tooth, covering the crown. It is a very hard, white to off-white, highly mineralised substance that acts as a barrier to protect the tooth but can become susceptible to degradation, especially by acids from food and drink. As disclosed herein, "dentine" is a calcified tissue of the body and, along with enamel, cementum, and pulp, is one of the four major components of teeth. It is usually covered by enamel on the crown and cementum on the root and surrounds the entire pulp. In a preferred embodiment, the bioceramic is obtained from shark teeth enameloid, from dentine or from a mixture of both enameloid and dentine. Both parts are mechanically separated to obtain two differentiated fractions as described (López-Alvarez et al, Biomed. Mater. 2016. 11:035011). Either each fraction, or the mixture of enameloid and dentine, is subjected afterwards to a sieving process by sieving the biomaterial.

In a preferred embodiment the bioceramic is obtained from the enameloid of teeth of the species *Prionace glauca.* In another embodiment the bioceramic is obtained from dentine of teeth of the species *Prionace glauca.* In another embodiment the bioceramic is obtained from a mixture of dentine and enameloid of teeth of the species *Prionace glauca.*

In a preferred embodiment the bioceramic is obtained from the enameloid of teeth of the species *Isurus oxyrinchus.* In another embodiment the bioceramic is obtained from dentine of teeth of the species *Isurus oxyrinchus.* In another embodiment the bioceramic is obtained from a mixture of dentine and enameloid of teeth of the species *Isurus oxyrinchus.*

In a preferred embodiment the bioceramic is obtained from a mixture of enameloid of teeth of the species *Prionace glauca* and enameloid of teeth of the species *Isurus oxyrinchus.* In another embodiment the bioceramic is obtained from a mixture of dentine of teeth of the species *Prionace glauca* and dentine of teeth of the species *Isurus oxyrinchus.* In another embodiment the bioceramic is obtained from a mixture of dentine and enameloid of teeth of the species *Prionace glauca* and of dentine and enameloid of teeth of the species *Isurus oxyrinchus.*

In a preferred embodiment the bioceramic is obtained from a mixture of enameloid of teeth of the species *Prionace glauca* and dentine of teeth of the species *Isurus oxyrinchus.* In a preferred embodiment the bioceramic is obtained from a mixture of enameloid of teeth of the species *Isurus oxyrinchus* and dentine of teeth of the species *Prionace glauca.*

As used herein, the terms "treatment", "treating" and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment," as used herein, covers any treatment of a disease in a mammal, particularly in a human, and includes: (a) increasing survival time; (b) decreasing the risk of death due to the disease; (c) preventing the disease from occurring in a subject which may be predisposed to the disease but has not yet been diagnosed as having it; (d) inhibiting the disease, i.e., arresting its development (e.g., reducing the rate of disease progression); and (e) relieving the disease, i.e., causing regression of the disease.

As disclosed herein, "hard tissue" (also termed "calcified tissue") is tissue which is mineralized and has a firm intercellular matrix. Non-limiting examples of hard tissue are bone, tooth enamel, dentine or cementum.

As disclosed herein, "injury" is any kind of damage to the body caused by an external force. Non-limiting examples of injury causing external forces are: accidents, falls, hits or weapons. As disclosed herein, "pathology" is any abnormal condition that affects part or all of an organism not caused by an external force and that consists of a disorder of a structure or function of the organism with certain symptoms and signs. Non-limiting examples of pathologies are: infections, caries, arthrosis, arthritis... As disclosed herein, the terms "injury" and "pathology" are not mutually excluding, meaning for example that an injury may afterwards cause a certain pathology. As disclosed herein the hard tissue injury or pathology can be any known in the art, including amongst others: defects, malformations, fractures, caries or infections.

In a preferred embodiment, the bioceramic is a granulate. As disclosed herein a "granulate" is a mixture of grains or granules within a range of different diameters. The granulate may be obtained as disclosed (López-Álvarez et al, Biomed. Mater. 2016, 11 035011). In a preferred embodiment, the granulate has a diameter size in a range selected from between 4 mm and below 20 µm. In a more preferred embodiment the granulate has a diameter size in a range selected from the group consisting of between 3.15 mm and 4 mm, between 2 mm and 3.15 mm, between 1 mm and 2 mm, between 0.5 mm and 1 mm, between 63 µm and 0.5 mm, between 20 µm and 63 µm and below 20 µm, or any combination of the upper and lower values of any of the ranges mentioned above. In a more preferred embodiment the granulate has a diameter size in a range of 2-3.15 mm, 1-2 mm and 0.5-1mm. In a still more preferred embodiment the granulate has a diameter size of 1mm.

In a preferred embodiment the bioceramic is obtained by a process comprising sieving the enameloid, the dentine or the mixture thereof to select granulates having a desired diameter and pyrolizing the resulting material to remove organic material. "Sieving" as disclosed herein is a simple technique for separating particles of different sizes. Depending upon the types of particles to be separated, sieves with different types of holes may be used. In a preferred embodiment, sieving is used to separate granulates obtained from the enameloid, the dentine or a mixture of both. In a preferred embodiment, sieves have a size of 4 mm, 3.15 mm, 2 mm, 1 mm, 0.5 mm, 63 µm or 20 µm. In a still more preferred embodiment, sieves have a size 1 mm and 0.5 mm.

As disclosed herein, "pyrolysis" means a thermal decomposition of materials at elevated temperatures, which is most commonly applied to the treatment of organic materials. "Organic matter" or "organic material" refers to the large pool of carbon-based compounds found within natural and engineered, terrestrial and aquatic environments. It is matter composed of organic compounds that has come from the remains of organisms such as plants and animals and their waste products in the environment. In an embodiment, pyrolysis is used to remove organic matter from shark teeth. In a preferred embodiment, pyrolysis is carried out in the range of 900 - 1150°C for 12 - 48 h. In a more preferred embodiment, pyrolysis is carried out at 950 °C for 12 h with a heating ramp of 2 °C min⁻¹ and a cooling ramp of 20 °C min⁻¹. In a preferred embodiment after pyrolysis the sieving process is repeated again to select granulates of a desired diameter range. Depending upon the types of particles to be separated, sieves with different types of holes may be used. In a preferred embodiment, sieves have a size of 4 mm, 3.15 mm, 2 mm, 1 mm, 0.5 mm, 63 µm or 20 µm. In a still more preferred embodiment, sieves have a size of 1 and 0.5 mm.

In a preferred embodiment the bioceramic comprises an apatitic crystalline phase and a non-apatitic crystalline phase. As disclosed herein, the apatitic phase comprises apatite. "Apatite" is a group of phosphate minerals, usually referring to hydroxyapatite (Ca₅(P0₄)₃0H) with CAS number 12167-74-7, fluorapatite (Ca₅(P0₄)₃F) with CAS number 1306-05-4 and chlorapatite (Ca₅(P0₄)₃Cl) with CAS number 1306-04-3, with high concentrations of OH⁻, F⁻ and Cl⁻ ions, respectively, in the crystal. The structure is crystallized in the monoclinic or hexagonal system. As disclosed herein, the crystalline non-apatitic phase is composed of tricalcium bis(orthophosphate) (Ca₃O₈P₂) (also refered as β-TCP) with CAS number 7758-87-4, whitlockite (Ca₉FeH₂MgO₃₂P₈) with CAS number 14358-97-5 or any combination thereof. In a preferred embodiment, stabilizing impurities can be present in β-TCP, preferably magnesium (Mg) or iron (Fe). In a preferred embodiment, β-TCP is stabilized with Mg. In another preferred embodiment, β-TCP is stabilized with Fe.

In a preferred embodiment, if the bioceramic has been obtained from enameloid, the crystalline apatitic phase is in higher percentage than the crystalline non-apatitic phase. In a more preferred embodiment, the enameloid crystalline apatitic phase is at least 51%, at least 60%, at least 70%, at least 80%, at least 90%, at least 100%, of the enameloid. In a still more preferred embodiment, the enameloid crystalline apatitic phase comprises about 91% apatitic phase and about 9% non-apatitic phase.

In another preferred embodiment, if the bioceramic has been obtained from dentine, the crystalline apatitic phase is in higher percentage than the crystalline non-apatitic phase. In a more preferred embodiment, the dentine crystalline apatitic phase is at least 51%, at least 60%, at least 70%, at least 80%, at least 90%, at least 100%, of the dentine. In a still more preferred embodiment, the dentine crystalline apatitic phase comprises about 63% apatitic phase and about 37% non-apatitic phase.

In another preferred embodiment, if the bioceramic has been obtained from a mixture of enameloid and dentine, the crystalline apatitic phase is in higher percentage than the crystalline non-apatitic phase. In a more preferred embodiment, the crystalline apatitic phase is at least 51%, at least 60%, at least 70%, at least 80%, at least 90%, at least 100%, of the mixture of enameloid and dentine. In a more preferred embodiment, the mixture of enameloid and dentine, comprises about 70% apatitic phase and about 30% non-apatitic phase.

In a preferred embodiment, if the bioceramic has been obtained from enameloid, the crystalline apatitic phase comprises fluoroapatite (Ca₅(PO₄)₃F) and the non-apatitic phase comprises β-TCP (Ca₃O₈P₂) and/or whitlockite (Ca₉Mg_{0.7}Fe²⁺_{0.5}(PO₄)₆(PO₃OH)).

In a another preferred embodiment, if the bioceramic has been obtained from dentine, the apatitic phase comprises hydroxyapatite (Ca₅(PO₄)₃OH) and/or apatite-(CaF) (H_{0.6}Ca₅F_{0.4}O_{12:6}P₃) and the non-apatitic phase comprises β-TCP (Ca₃O₈P₂) and/or whitlockite (Ca₉Mg_{0.7}Fe²⁺_{0.5}(PO₄)₆(PO₃OH).

In another preferred embodiment, if the bioceramic has been obtained from a mixture of enameloid and dentine then the apatitic phase comprises hydroxyapatite Ca₅(PO₄)₃OH), and/or apatite-(CaF) (H_{0.6}Ca₅F_{0.4}O_{12:6}P₃)) and/or a fluorapatite (Ca₅(PO₄)₃F), and the non-apatitic phase comprises β-TCP (Ca₃O₈P₂) and/or whitlockite (Ca₉Mg_{0.7}Fe²⁺_{0.5}(PO₄)₆(PO₃OH).

As disclosed herein, the hard tissue to be treated according to the invention is bone or dental tissue. In a preferred embodiment, the hard tissue belongs to any animal classified as mammals and includes, but is not restricted to the Order Rodentia, such as mice; Order Logomorpha, such as rabbits; more particularly the Order Carnivora, including Felines (cats) and Canines (dogs); even more particularly the Order Artiodactyla, Bovines (cows) and Suines (pigs); and the Order Perissodactyla, including Equines (horses); and most particularly the Order Primates, Ceboids and Simoids (monkeys) and Anthropoids (humans and apes). The mammals of preferred embodiments are humans.

Non-limiting examples of medical fields wherein the bioceramic according to the invention can be used include: odontology, traumatology, orthopedic surgery, maxilofacial surgery and otorhinolaryngology.

Non-limiting examples of use of the bioceramic according to the invention in odontology include: implantology, periodontics and general surgery. Treatments used in implantology include, without limitation: treatment of peri-implant bone defects (both during the implant placement and in the treatment of complications, such as peri-implantitis), maxillary sinus lift (to achieve bone height for place implants in the posterior region of the upper jaw), horizontal increase of a narrow bone crest or vertical increase of the bone crest (with blocks of bone substitute). Treatments used in periodontics include, without limitation: treatment of intrabone defects (loss of bone around different areas of the root of a tooth, usually due to the periodontal disease) or treatment of furcation defects (loss of bone between the root of a molar). Treatments used in general oral surgery include, without limitation: filling of alveoli after extraction (which allows to keep in height and width the alveolar bone ridge for a posterior placement of dental prosthesis, both conventional and implant-supported) or apicectomies (filling of the periapical bone cavity after the removal of a periapical granuloma).

Non-limiting examples of use of the bioceramic according to the invention in traumatology and surgery include: articular fractures, open fractures with bone loss, tumor surgery, orthopedic surgery or spine surgery.

Use in traumatology: in articular fractures due to compression mechanisms may produce a variable degree of subsidence (depending on the intensity of the trauma) of the cartilaginous articular surface and the underlying subchondral bone in metaphyseal cancellous bone. When the fragments rise again to the original height of the joint surface during the surgical treatment, a defect is created as a result of the compaction suffered by the cancellous bone. To provide mechanical strength to the reconstruction and internal fixation, this defect must be filled. Spontaneous autologous or homologous bone graft can be used, but bone substitutes of non-bone origin are usually used. It can also be used with open fractures with bone loss, in the absence of infection. They are sometimes used to supplement the fixation with internal fixation devices when the bone is inconsistent as it is in fractures in very osteoporotic bone.

Use in tumor surgery: in the majority of pseudotumoral and benign tumor lesions that develop in the bones, an intralesional treatment consisting of curettage of the lesion, milling of the walls, some local coadjuvant treatment (phenol, liquid nitrogen, etc.) and subsequent filling with bone substitutes of bone origin, combined or exclusively also with substitutes of non-bone origin.

Use in orthopedic surgery: for the non-prosthetic treatment of knee arthropathies that selectively affect the medial compartment of the knee, valgusant osteotomies of medial opening are made in the proximal metaphysis of the tibia and the bone fragments are fixed with a plate and screws. The resulting wedge-shaped bone defect at that level can also be filled with bone substitutes of non-bone origin. It can also be used for total hip and knee arthroplasty. As a consequence of the inflammatory reaction secondary to the wear particles generated at the level of the mobile surfaces (especially in the Metal-UHMWPE friction torques), areas of osteolysis occur around the metallic implants. These areas can and usually are filled with non-bone substitutes. This occurs most frequently at the retroacetabular level and the proximal femur in the case of total hip arthroplasty. In these cases it is not necessary to provide mechanical strength. It is simply a matter of filling the cavities to try to restore bone stock.

As a result of the failures of prosthetic knee implants, especially, arthrodesis of the joint is indicated with some frequency. In these cases there are always serious bone defects. In addition to the stabilization of the joint with internal or external fixation, the uninfected residual cavities are filled with bone grafts combined with substitutes of non-bone origin, sometimes also with cement (PMMA).

Use in spinal surgery: to achieve intersomatic and circumferential arthrodesis.

Non-limiting examples of use of the bioceramic according to the invention in maxilofacial surgery and otorhinolaryngology include: ear and maxillofacial surgery, cases of filling of cavities produced by surgical obliteration (mastoids, sinuses, external table, ...) or tiroplasty, wedge of hydroxyapatite to move vocal cord.

In a preferred embodiment, the hard tissue injury or pathology is an articular defect. "An articulation" or "joint" as disclosed herein is the connection made between bones in the body which link the skeletal system into a functional whole, allowing for different degrees and types of movement. Non-limiting examples of articulations are knee, elbow, wrist, ankle or shoulder. According to the type of binding tissue that connects the bones to each other, articulations or joints may be classified as:
- fibrous joint: joined by dense regular connective tissue that is rich in collagen fibers;
- cartilaginous joint: joined by cartilage. There are two types: primary cartilaginous joints composed of hyaline cartilage, and secondary cartilaginous joints composed of hyaline cartilage covering the articular surfaces of the involved bones with fibrocartilage connecting them;
- synovial joint (not directly joined): the bones have a synovial cavity and are united by the dense irregular connective tissue that forms the articular capsule that is normally associated with accessory ligaments;
- facet joint: joint between two articular processes between two vertebrae.

As disclosed herein, "an articular defect" may be any kind of damage to the articulation, either in the bone or in the binding tissue, which prevents the articulation from performing its normal movement.

In another preferred embodiment, the hard tissue injury or pathology is a bone fracture or a bone defect. As disclosed herein, a "bone fracture" is a medical condition in which there is damage in the continuity of the bone. A bone fracture may be the result of high force impact or stress, or a minimal trauma injury as a result of certain medical conditions that weaken the bones, such as osteoporosis, bone cancer, or osteogenesis imperfecta. As disclosed herein, a "bone defect" is a lack of bone where it should normally occur. Bone defects may be caused by trauma, tumor, or infection (osteomyelitis). Bone defects can be repaired by surgical reconstruction. Since bone defects arise from trauma or debridement for the treatment of osteomyelitis, the bone defects often accompany major soft-tissue (muscles, tendons, joints, etc.) injury.

In a preferred embodiment, the hard tissue injury or pathology is repaired by "arthrodesis", understood as the artificial induction of joint ossification between two bones by surgery. This is usually done to relieve intractable pain in a joint which cannot be managed by pain medication, splints, or other normally indicated treatments. Non-limiting examples of typical causes of such pain are fractures which disrupt the joint, severe sprains, and arthritis.

In a preferred embodiment, the hard tissue injury is repaired by "bone replacement" or "bone graft", which is especially relevant for repairing cranio- and maxillofacial defects, periodontal defects, bone fractures and other dental and orthopedic defects. Bone graft is a surgical procedure that replaces missing bone in order to repair bone fractures that are extremely complex, pose a significant health risk to the patient, or fail to heal properly. Bone grafts may be autologous (bone harvested from the patient's own body, often from the iliac crest), allograft (cadaveric bone usually obtained from a bone bank), or synthetic (often made of hydroxyapatite or other naturally occurring and biocompatible substances) with similar mechanical properties to bone. In a more preferred embodiment, the bone graft is synthetic.

In a preferred embodiment the hard tissue pathology is dental caries. "Dental caries" as used herein is a breakdown of teeth due to acids made by bacteria. The cavities may be a number of different colors from yellow to black. Symptoms may include pain and difficulty with eating. Complications may include inflammation of the tissue around the tooth, tooth loss, and infection or abscess formation. In a particular embodiment the cause of caries is acid from bacteria dissolving the hard tissues of the teeth (enamel, dentine and cementum). The acid is produced by the bacteria when they breakdown food debris or sugar on the tooth surface.

In a preferred embodiment the hard tissue injury or pathology is caused by an infection. The term "infection", as used herein, relates to invasion by bacteria, viruses, fungi, protozoa or other microorganisms, referring to the undesired proliferation or presence of pathogenic microbes in a host organism. It includes the excessive growth of microbes that are normally present in and/or on the body of a mammal or other organism. More generally, a microbial infection can be any situation in which the presence of a microbial population(s) is damaging to a host mammal. Thus, a microbial infection exists when excessive numbers of a microbial population are present in and/or on a mammal's body, or when the effects of the presence of a microbial population(s) is damaging the cells or other tissue of a mammal. In a preferred embodiment, the infection is developed after surgery. In a more preferred embodiment, the infection is developed after surgery in the ear or in the face.

In a preferred embodiment, the infection is a bacterial infection, caused by bacteria of any species. In a more preferred embodiment, the infection is caused by one or more of a bacterial species selected from Gram-positive bacteria. In a more preferred embodiment, bacteria are Gram-positive cocci. In a more preferred embodiment bacteria belong to *Staphylococcus* genus. In a still more preferred embodiment, bacteria are *Staphylococcus aureus* and/or *Staphylococcus epidermidis.*

In a preferred embodiment, the bacterial infection is caused by a biofilm. A "biofilm" as disclosed herein comprises any group of microorganisms in which cells stick to each other and often also to a surface. In a still more preferred embodiment, the biofilm grows on the surface of the injured tissue and/or of the tissue surrounding the injured tissue. As used herein, the term "injured" is used in its ordinary sense to refer to any tissue damage including a wound, trauma or lesion or any tissue degeneration.

In a preferred embodiment, the environment in or in the proximity of the hard tissue to be treated is acidic or prone to become acidic, or wherein the hard tissue is in contact with an acidic fluid. As disclosed herein, "environment" is everything that surrounds the hard tissue in a living organism. In a preferred embodiment, the hard tissue is bone or dental tissue. In a preferred embodiment, the environment in or in the proximity of the hard tissue to be treated is acidic or the hard tissue is in contact with an acidic fluid. "Acidic" as disclosed herein is an environment wherein the pH is below 7. In a preferred embodiment the pH is lower than 7.0. In a more preferred embodiment, the pH is between 6.0 and 4.0. In a still more preferred embodiment, the pH is below 5.5. In a preferred embodiment when the hard tissue is dental tissue the acidic fluid is saliva. In saliva the pH may be below the critical pH, which is the pH at which saliva and plaque fluid cease to be saturated with calcium and phosphate, thereby permitting the hydroxyapatite in dental enamel to dissolve. In a preferred embodiment, saliva's low pH causes caries.

In a preferred embodiment, the bioceramic according to the invention can be impregnated with an antiseptic or an antibiotic. The term "antibiotic", as used herein, relates to a chemical substance produced by a living being or a synthetic derivative thereof which at low concentrations kills or prevents the growth of certain classes of sensitive microorganisms, generally bacteria, although some antibiotics are also used for the treatment of infections by fungi or protozoa. Antibiotics are used in human, animal or horticultural medicine to treat infections caused by microorganisms. Antibiotics included in the present invention are, without limitation, aminoglycoside antibiotics, ansamycins, carbacefem, carbapenems, cephalosporins, glycopeptides, macrolides, monobactams, penicillins, polypeptides, quinolones, sulfonamides, tetracyclines and others such as arsphenamine, chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin/dalfopristin, rifampin or rifampicin, tinidazole, viomycin and capreomycin; preferably cephalosporins, tetracyclines, glycopeptides, carbapenems, polypeptides, rifampicin, aminoglycosides, sulfonamides, viomycin and capreomycin. In a preferred embodiment the antibiotic is selected from the group of carbapenems, cephalosporins, monobactams, penicillins, polypeptides, quinolones, sulfonamides and tetracyclines.

As used herein, the term "antiseptic" refers to a chemical agent that kills or prevents the growth of pathogenic or non-pathogenic bacteria. Antiseptics include without limitation: chlorhexidine, alcohols, hydrogen peroxide or iodine.

### Oral composition comprising a bioceramic and its use in the treatment or prevention of a hard tissue injury or pathology

The inventors of the present invention have found that the bioceramic according to the invention can be used in an oral composition such as a dentifrice. Therefore, in another aspect the invention relates to an oral composition comprising a bioceramic according to the invention. Oral composition may be any of the following oral compositions selected from the group consisting of: a toothpaste or a dentifrice, a mouthwash or a mouth rinse, a topical oral gel, a chewing gum, and a denture cleanser.

As disclosed herein, a "dentifrice" is an agent that can be used along with a toothbrush to clean and polish natural teeth, which includes toothpowder and toothpaste. A dentifrice can be supplied in any format known in the art, such as a paste, powder, gel or liquid form. Commonly known dentifrices include toothpaste, mouthwash, chewing gum, dental floss, and dental cream. Other examples of dentifrices include toothpowder, mouth detergent, troches, dental or gingival massage cream, dental strips, dental gels, and gargle tablets. The dentifrice composition can be in any desired form such as deep striped, surface striped, multi-layered, having the gel surrounding the paste, or any combination thereof. Alternatively the oral composition is provided as a dual phase composition, wherein individual compositions are combined when dispensed from a separated compartment dispenser.

As disclosed herein, a "mouthwash" is a liquid which is held in the mouth passively or swilled around the mouth by contraction of the perioral muscles and/or movement of the head, and may be gargled, where the head is tilted back and the liquid bubbled at the back of the mouth. Usually mouthwashes are antiseptic solutions intended to reduce the microbial load in the oral cavity, although other mouthwashes might be given for other reasons such as for their analgesic, anti-inflammatory or antifungal action. Additionally, some rinses act as saliva substitutes to neutralize acid and keep the mouth moist in xerostomia (also known as "dry mouth" and "dry mouth syndrome" is dryness in the mouth, which may be associated with a change in the composition of saliva, or reduced salivary flow, or have no identifiable cause. Risk factors including conditions that result in less saliva include diabetes mellitus, Sjögren's syndrome or certain medications, amongst others.). Cosmetic mouthrinses temporarily control or reduce bad breath and leave the mouth with a pleasant taste.

Oral compositions according to the invention may comprise, in addition to the bioceramic material disclosed herein, one or more of the following:
- One or more sources of zinc,
- One or more polyphosphates,
- One or more copolymers
- Glycerin
- One or more whitening agents,
- One or more thickening agents
- One or more flavoring agents
- One or more surfactants
- One or more sweetening agents
- One or more anti-caries agents.

In a preferred embodiment the dentifrice is used in the treatment or prevention of dental caries.

In another preferred embodiment the dentifrice is used in the treatment of enamel remineralization and/or to inhibit dental sensitivity. As disclosed herein, "enamel remineralization" is defined as a process in which calcium and phosphate ions are sourced to promote ion deposition into crystal voids in demineralized enamel. Remineralization remains imperative towards the management of non-cavitated carious lesions and prevention of disease progression within the oral cavity. The process also has the ability to contribute towards restoring strength and function within tooth structure. As disclosed herein, "dental sensitivity" is defined as intense and transitory pain that is caused by the exposure of the dentine, the internal part of teeth, to the oral environment and which occurs when contact is made with an external stimulus: food or drink that is cold, hot, acidic, sweet; tactile pressure, etc.

All the terms and embodiments previously described are equally applicable to this aspect of the invention.

### Additional aspects of the invention:

1. A bioceramic obtained from shark teeth for use in the treatment or prevention of a hard tissue injury or pathology.
2. The bioceramic for use according to aspect 1 wherein the teeth are from *Prionace glauca* and/or *Isurus oxyrinchus.*
3. The bioceramic for use according to aspects 1 or 2, wherein the bioceramic is obtained from teeth enameloid, from teeth dentine or from a mixture of teeth enameloid and teeth dentine.
4. The bioceramic for use according to any of aspects 1 to 3 wherein the bioceramic is a granulate having a size selected from the group consisting of between 3.15 mm and 4 mm, between 2 mm and 3.15 mm, between 1 mm and 2 mm, between 0.5 mm and 1 mm, between 63 µm and 0.5 mm, between 20 µm and 63 µm and below 20 µm.
5. The bioceramic for use according to aspects 1-4 wherein the bioceramic is obtained by a process comprising sieving the enameloid, the dentine or the mixture thereof to select granulates having a desired diameter and pyrolizing the resulting material to remove organic material.
6. The bioceramic for use according to aspects 1-5 wherein the pyrolysis step is followed by a second sieving process to select granulates having the desired diameter.
7. The bioceramic for use according to any one of aspects 1-6, wherein the bioceramic comprises an apatitic crystalline phase and a non-apatitic crystalline phase.
8. The bioceramic for use according to any one of aspects 1-7, wherein the apatitic crystalline phase comprises apatite, fluorapatite, hydroxyapatite or any combination thereof.
9. The bioceramic for use according to any one of aspects 1 to 7, wherein the non-apatitic crystalline phase comprises whitlockite, tricalcium ortho-phosphate (β-TCP) or a combination thereof.
10. The bioceramic for use according to any one of aspects 1-9, wherein
   (i) If the bioceramic has been obtained from enameloid, it comprises about 91% apatitic phase and about 9% non-apatitic phase,
   (ii) If the bioceramic has been obtained from dentine, it comprises about 63% apatitic phase and about 37% non-apatitic phase and
   (iii) If the bioceramic has been obtained from a mixture of enameloid and dentine, it comprises about 70% apatitic phase and about 30% non-apatitic phase.
11. The bioceramic for use according to aspect 10 wherein
   (i) If the bioceramic has been obtained from enameloid and it comprises about 91% apatitic phase and about 9% non-apatitic phase, then the apatitic phase comprises fluoroapatite (Ca₅(PO₄)₃F) and the non-apatitic phase comprises β-TCP (Ca₃O₈P₂) and/or whitlockite (Ca₉Mg_{0.7}Fe²⁺_{0.5}(PO₄)₆(PO₃OH)),
   (ii) If the bioceramic has been obtained from dentine and it comprises about 63% apatitic phase and about 37% non-apatitic phase, then the apatitic phase comprises hydroxyapatite (Ca₅(PO₄)₃OH) and/or apatite-(CaF) (H_{0.6}Ca₅F_{0.4}O_{12:6}P₃) and the non-apatitic phase comprises β-TCP (Ca₃O₈P₂) and/or whitlockite (Ca₉Mg_{0.7}Fe²⁺_{0.5}(PO₄)₆(PO₃OH) and
   (iii) If the bioceramic has been obtained from a mixture of enameloid and dentine and it comprises about 70 % apatitic phase and about 30% non-apatitic phase, then the apatitic phase comprises hydroxyapatite Ca₅(PO₄)₃OH), and/or apatite-(CaF) (H_{0.6}Ca₅F_{0.4}O_{12:6}P₃)) and/or a fluorapatite (Ca₅(PO₄)₃F), and the non-apatitic phase comprises β-TCP (Ca₃O₈P₂) and/or whitlockite (Ca₉Mg_{0.7}Fe²⁺_{0.5}(PO₄)₆(PO₃OH).
12. The bioceramic for use according to any one of aspects 1 to 11, wherein the hard tissue is bone or dental tissue.
13. The bioceramic for use according to any one of aspects 1 to 12, wherein the hard tissue injury or pathology is an articular defect, a bone fracture or a bone defect.
14. The bioceramic for use according to any one of aspects 1 to 13, wherein the hard tissue injury or pathology is repaired by arthrodesis.
15. The bioceramic for use according to any one of aspects 1 to 14, wherein the bone fracture or defect is repaired by bone replacement.
16. The bioceramic for use according to any one of aspects 1 to 15, wherein the hard tissue pathology is dental caries.
17. The bioceramic for use according to any one of aspects 1 to 12, wherein the hard tissue injury or pathology is caused by an infection.
18. The bioceramic for use according to aspect 17 wherein the infection is caused by a bacterial infection.
19. The bioceramic for use according to aspects 17 or 18, wherein the infection is caused by a bacterial biofilm.
20. The bioceramic for use according to any one of aspects 17 to 19, wherein the bacterial biofilm grows on the surface of the hard tissue.
21. The bioceramic for use according to any one of aspects 1 to 20, wherein the environment in or in the proximity of the hard tissue to be treated is acidic or prone to become acidic, or wherein the hard tissue is in contact with an acidic fluid.
22. The bioceramic for use according to any one of aspects 1-21, wherein the hard tissue is dental tissue and wherein the acidic fluid is saliva.
23. The bioceramic for use according to aspects 21 or 22, wherein the pH of the acidic environment is between 4 and 6.
24. The bioceramic for use according to any one of aspects 1 to 23, wherein the surface of the bioceramic is impregnated with an antiseptic or an antibiotic.
25. An oral composition comprising a bioceramic as defined in any of aspects 1 to 11.
26. The oral composition according to aspect 25 which is a toothpaste or a dentifrice, a mouthwash or a mouth rinse, a topical oral gel, a chewing gum, and a denture cleanser
27. The oral composition according to aspects 25 or 26 for use in the treatment or prevention of dental caries.
28. The oral composition according to aspects 25 or 26 for use in the treatment of enamel remineralization and/or to inhibit dental sensitivity.

The invention is described below by way of the following examples which are to be construed as merely illustrative and not limitative of the scope of the invention.

### EXAMPLES

### Study of the stability of the bioceramic obtained from shark tooth (Prionace glauca and/or Isurus oxyrinchus) enamel, dentine and a mixture of shark tooth in an acidic medium

### Materials and methods

### Preparation of a bioceramic from shark teeth enameloid

*Isurus oxyrinchus* and *Prionace glauca* shark teeth were provided by the Centro Tecnológico del Mar (CETMAR, Vigo, Spain) and by the fishing company COPEMAR S.A. (Porto de Vigo, Spain). After washing in boiling water for 3 h to separate the organic remains of the sharks' teeth and their drying in a laboratory oven at 60 °C for 24 h, the two sections of teeth, the enameloid and dentine, were mechanically separated to obtain two differentiated fractions. Then, the enameloid fraction was grinded and subjected to a sieving process (with sieves of 4 mm, 1 mm, 63 *µ*m and 20 *µ*m) to select granules in the diameter range of 1 - 2 mm, and then pyrolyzation at 950 °C for 12 h with a heating ramp of 2 °C min⁻¹ and a cooling ramp of 20 °C min⁻¹ to remove the organic matter.

### Preparation of a bioceramic from shark teeth dentine

*Isurus oxyrinchus* and *Prionace glauca* shark teeth were provided by the Centro Tecnológico del Mar (CETMAR, Vigo, Spain) and by the fishing company COPEMAR S.A. (Porto de Vigo, Spain). After washing in boiling water for 3 h to separate the organic remains of the sharks' teeth and their drying in a laboratory oven at 60 °C for 24 h, the two sections of teeth, the enameloid and dentine, were mechanically separated to obtain two differentiated fractions. Then, the dentine fraction was grinded and subjected to a sieving process (with sieves of 4 mm, 1 mm, 63 *µ*m and 20 *µ*m) to select granules in the diameter range of 1 - 2 mm, and then pyrolyzation at 950 °C for 12 h with a heating ramp of 2 °C min⁻¹ and a cooling ramp of 20 °C min⁻¹ to remove the organic matter.

### Preparation of a bioceramic from a mixture of shark teeth enameloid and dentine

*Isurus oxyrinchus* and *Prionace glauca* shark teeth were provided by the Centro Tecnológico del Mar (CETMAR, Vigo, Spain) and by the fishing company COPEMAR S.A. (Porto de Vigo, Spain). After washing in boiling water for 3 h to separate the organic remains of the sharks' teeth and their drying in a laboratory oven at 60 °C for 24 h, the teeth were grinded. Then, the mixture of shark teeth enameloid and dentine fraction was subjected to a sieving process (with sieves of 4 mm, 1 mm, 63 *µ*m and 20 *µ*m) to select granules in the diameter range of 1 - 2 mm , and then pyrolyzation at 950 °C for 12 h with a heating ramp of 2 °C min⁻¹ and a cooling ramp of 20 °C min⁻¹ to remove the organic matter.

### Stability assay under acidic conditions

Bioceramic obtained from shark tooth (*Prionace glauca* or *Isurus oxyrinchus)* enamel, dentine and a mixture of shark tooth with a grain size of 1-2 mm in diameter were incubated in citrate buffer pH 4.0, 4.5, 5.0, and 6.0 (mass:volume ratio of 1 mg:1 ml) for 1 hour at 37°C under stirring conditions (20 rpm). Granules with a 1-2 mm diameter from a commercial synthetic biphasic bone filler based on a composite with 40% tricalcium phosphate and 60% hydroxyapatite (Bi-Ostetic™, Berkeley Advanced Biomaterials Inc.) and from the bio-derived bone filler of a trabecular porous bone mineral matrix produced from bovine bone with 0.5 - 1 mm diameter (Bio-Oss®, Geistlich Biomaterials) were also tested.

Three replicates per condition were performed for all of them. After the immersion period has elapsed, 1.5 ml were collected from each container for evaluating the Ca, P, and Mg ions dissolved in the citrate buffer at different pHs by means of the ICP-OES technique (CACTI, University of Vigo). To that end, a 1:5 dilution was first performed in 2% nitric acid. Indium, the internal standard of the equipment itself, was used as a reference standard.

### Results

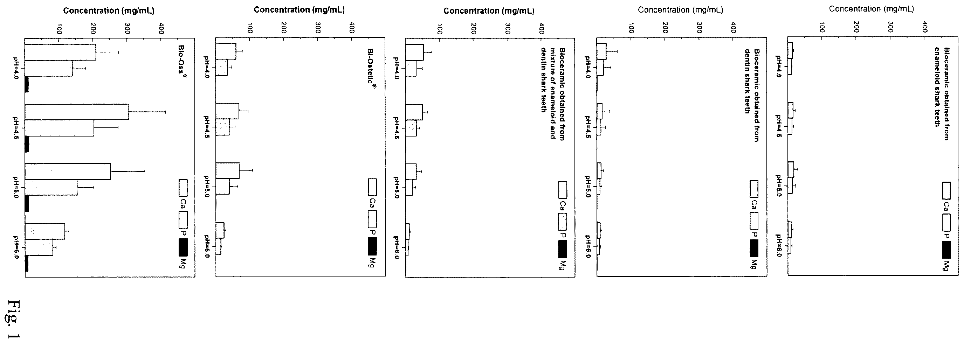

Figure 1 shows five histograms depicting the concentration of Ca, P, and Mg (mg/l) corresponding to each volume collected after immersion of the different materials (bioceramic obtained from shark tooth enamel, from shark tooth dentine, from shark tooth containing both enamel and dentine, Bio-Oss®, and Bi-Ostetic®) in different citrate buffers (pH=4.0, 4.5, 5.0, and 6.0). When evaluating the results that are obtained, it can be clearly seen that the tested material that is the most stable at the different acidic pHs was the bioceramic obtained from shark tooth enamel, while the most unstable one which has a high degree of Ca, P, and Mg dissolution was the commercial material, Bio-Oss®. Finally, the bioceramic obtained from from shark tooth dentine, a mixture of shark tooth, and the synthetic material Bi-Ostetic®, were ranked between the most stable and the most unstable. Focusing on the dissolved Ca, for the same material mass and under the same conditions at a pH of 4.5 (below the critical pH), the dissolved amount of Bi-Ostetic® and the dissolved amount of Bio-Oss® are, respectively, 4.18-fold and 18.67-fold greater in comparison with the bioceramic obtained from shark tooth enamel.

A linear relationship between dissolution and pH was not observed, where the pH remains virtually constant in the case of the bioceramic obtained from shark tooth enamel. Dissolution clearly increases as the pH drops below 6.0 both for Bi-Ostetic® and Bio-Oss®, but the values remain constant between pH 5.0, 4.5, and 4.0, particularly in Bi-Ostetic®, a greater variability being observed in Bio-Oss® in terms of mean value but, taking into account the error bars, those variations are not statistically significant.

It is demonstrated that the bioceramic obtained from shark tooth enamel is the most stable bioceramic in an acidic environment in the pH range between 6.0 and 4.0, followed by the bioceramic obtained from dentine and a mixture (enamel and dentine) of shark tooth. In contrast, the control materials (Bi-Ostetic® and Bio-Oss®) have significant dissolution rates in an acidic environment.

### Proof of concept in veterinary medicine

Species: Mongrel dog with high activity level.

Pathology: Complex deformation of the forelimbs. Severe radiocarpal joint injuries.

Materials and methods: Metal joint fusion implant and bioceramic obtained from shark tooth (*Prionace glauca* and/or *Isurus oxyrinchus).* A joint fusion is performed to stabilize the damaged joint and recover natural support.

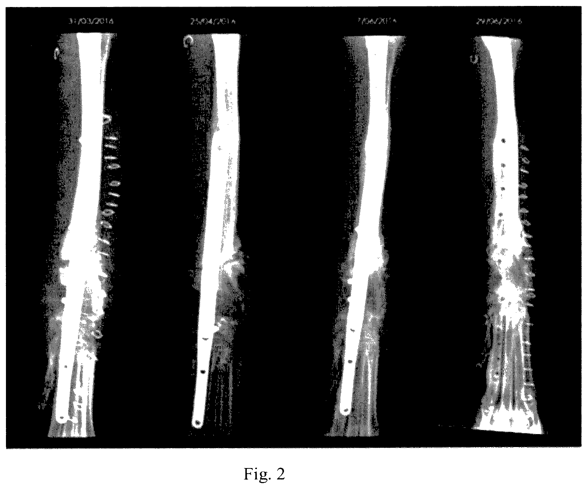

Results: The use of the bioceramic obtained from shark tooth (*Prionace glauca* and/or *Isurus oxyrinchus*) provided a quick and solid fusion. As can be seen in Figure 2, the use of this bioceramic allowed removing the joint fusion implant earlier than usual and starting rehabilitation. The recovery time was thereby significantly reduced.

## Claims

1. A bioceramic obtained from shark teeth for use in the treatment or prevention of a hard tissue injury or pathology.

2. The bioceramic for use according to claim 1, wherein the bioceramic is obtained from teeth enameloid, from teeth dentine or from a mixture of teeth enameloid and teeth dentine.

3. The bioceramic for use according to claims 1 or 2, wherein the bioceramic comprises an apatitic crystalline phase and a non-apatitic crystalline phase.

4. The bioceramic for use according to any one of claims 1-3, wherein
(i) If the bioceramic has been obtained from enameloid, it comprises about 91% apatitic phase and about 9% non-apatitic phase,
(ii) If the bioceramic has been obtained from dentine, it comprises about 63% apatitic phase and about 37% non-apatitic phase and
(iii) If the bioceramic has been obtained from a mixture of enameloid and dentine, it comprises about 70% apatitic phase and about 30% non-apatitic phase.

5. The bioceramic for use according to claim 4 wherein
(i) If the bioceramic has been obtained from enameloid and it comprises about 91% apatitic phase and about 9% non-apatitic phase, then the apatitic phase comprises fluoroapatite (Ca₅(PO₄)₃F) and the non-apatitic phase comprises β-TCP (Ca₃O₈P₂) and/or whitlockite (Ca₉Mg_{0.7}Fe²⁺₀.₅(PO₄)₆(PO₃OH)),
(ii) If the bioceramic has been obtained from dentine and it comprises about 63% apatitic phase and about 37% non-apatitic phase, then the apatitic phase comprises hydroxyapatite (Ca₅(PO₄)₃OH) and/or apatite-(CaF) (H_{0.6}Ca₅F_{0.4}O_{12:6}P₃) and the non-apatitic phase comprises β-TCP (Ca₃O₈P₂) and/or whitlockite (Ca₉Mg_{0.7}Fe²⁺_{0.5}(PO₄)₆(PO₃OH) and
(iii) If the bioceramic has been obtained from a mixture of enameloid and dentine and it comprises about 70 % apatitic phase and about 30% non-apatitic phase, then the apatitic phase comprises hydroxyapatite Ca₅(PO₄)₃OH), and/or apatite-(CaF) (H_{0.6}Ca₅F_{0.4}O_{12:6}P₃)) and/or a fluorapatite (Ca₅(PO₄)₃F), and the non-apatitic phase comprises β-TCP (Ca₃O₈P₂) and/or whitlockite (Ca₉Mg_{0.7}Fe²⁺_{0.5}(PO₄)₆(PO₃OH).

6. The bioceramic for use according to any one of claims 1 to 5, wherein the hard tissue is bone or dental tissue.

7. The bioceramic for use according to any one of claims 1 to 6, wherein the hard tissue pathology is dental caries.

8. The bioceramic for use according to any one of claims 1 to 6, wherein the hard tissue injury or pathology is caused by an infection.

9. The bioceramic for use according to claim 8 wherein the infection is caused by a bacterial infection.

10. The bioceramic for use according to any one of claims 1 to 9, wherein the environment in or in the proximity of the hard tissue to be treated is acidic or prone to become acidic, or wherein the hard tissue is in contact with an acidic fluid.

11. The bioceramic for use according to any one of claims 1-10, wherein the hard tissue is dental tissue and wherein the acidic fluid is saliva.

12. The bioceramic for use according to claims 10 or 11, wherein the pH of the acidic environment is between 4 and 6.

13. An oral composition comprising a bioceramic as defined in any of claims 1 to 5.

14. The oral composition according to claim 13 for use in the treatment or prevention of dental caries.

15. The oral composition according to claims 13 or 14 for use in the treatment of enamel remineralization and/or to inhibit dental sensitivity.
